# EUROPÄISCHE PATENTSCHRIFT

(11) **EP 1 383 507 B1**
(45) Veröffentlichungstag und Bekanntmachung des Hinweises auf die Patenterteilung: **14.11.2007**
(21) Anmeldenummer: 02764017.6
(22) Anmeldetag: 18.04.2002
(51) Int. Cl.: A61K 31/55, A61P 25/28, A61P 25/30, A61P 25/32, A61P 25/34, A61P 25/36

(54) **VERWENDUNG VON GALANTHAMIN ZUR BEHANDLUNG VON KRANKHEITSERSCHEINUNGEN DES ZENTRALEN NERVENSYSTEMS AUFGRUND VON INTOXIKATIONEN MIT PSYCHOTROPEN SUBSTANZEN**
UTILIZATION OF GALANTHAMINE FOR THE TREATMENT OF PATHOLOGIES OF THE CENTRAL NERVOUS SYSTEM OWING TO INTOXICATIONS WITH PSYCHOTROPIC SUBSTANCES
UTILISATION DE GALANTHAMINE POUR TRAITER DES SYMPTOMES DU SYSTEME VERVEUX CENTRAL PROVOQUES PAR DES INTOXICATIONS DUES A DES SUBSTANCES PSYCHOTROPES

(30) Priorität: 24.04.2001 DE 10119862
(43) Veröffentlichungstag der Anmeldung: 28.01.2004
(73) Patentinhaber: HF ARZNEIMITTELFORSCHUNG GmbH, 59368 Werne (DE)
(72) Erfinder: OPITZ, Klaus, 48157 Münster (DE); MOORMANN, Joachim, 59368 Werne (DE); HILLE, Thomas, 56567 Neuwied (DE); BECHER, Frank, 56072 Koblenz (DE)
(74) Vertreter: Flaccus, Rolf-Dieter
(86) Internationale Anmeldenummer: PCT/EP2002/004277
(87) Internationale Veröffentlichungsnummer: WO 2002/085370

(56) Entgegenhaltungen:
- EP-A- 0 449 247
- WO-A-92/20328
- WO-A-94/16708
- US-A- 5 519 017
- STUNTZ P M ET AL: "Cholinergic stimulation enhances cognitive performance following cingulate cortex lesions in rats." SOCIETY FOR NEUROSCIENCE ABSTRACTS, Bd. 26, Nr. 1-2, 2000, Seiten Abstract No.-753.11, XP001094738 30th Annual Meeting of the Society of Neuroscience;New Orleans, LA, USA; November 04-09, 2000 ISSN: 0190-5295
- POWERS J S ET AL: "PHYSOSTIGMINE FOR TREATMENT OF DELIRIUM TREMENS" JOURNAL OF CLINICAL PHARMACOLOGY, LIPPINCOTT CO, HAGERSTOWN, MD, US, Bd. 21, Nr. 1, 1981, Seiten 57-60, XP000612564 ISSN: 0091-2700

## Beschreibung

Die Erfindung betrifft die Verwendung von Galanthamin zur Herstellung eines Arzneimittels zur Behandlung von kognitiven Störungen, wie eines allgemeinen Kontrollverlustes, des Gedächtnisverlustes oder einer Beeinträchtigung der Gedächtnisleistung, des Denkvermögens, von Demenz, von Wahrnehmungsstörungen, oder einer Beeinträchtigung der Bewegungskoordination, die infolge eines gelegentlichen oder chronischem Gebrauchs oder Mißbrauchs von halluzinogenen Substanzen, bei einer akuten Vergiftung oder bei chronischer Einwirkung von Umweltgiften beim Menschen oder anderen Wirbeltieren auftreten.

Die Zuführung von psychotropen Substanzen, auch von Rauschmitteln, insbesondere von Alkohol, führt bekanntermaßen zu Symptomen wie Wahrnehmungsstörungen, Gedächtnisverlust, Beeinträchtigung kognitiver Fähigkeiten, allgemeinem Kontrollverlust, Aggressivität, Beeinträchtigung der Bewegungskoordination etc..

Wird die Substanz als Rauschmittel, wie z. B. Heroin, Kokain oder als ethylalkoholhaltiges Getränk, bewußt zugeführt, so sind derartige Wirkungen zwar von der rauschmittelkonsumierenden Person beabsichtigt, jedoch werden sie unter bestimmten Voraussetzungen auch als nachteilig empfunden. Hinzu kommt, daß der Schweregrad und die zeitliche Fortdauer dieser Symptome variieren kann und für den Rauschmittelkonsumenten im voraus oft nur schwer einzuschätzen ist.

Insbesondere bei chronischer Abhängigkeit und fortgesetztem Suchtmittelmißbrauch kommt es nicht nur zu den allgemein bekannten Organschädigungen, sondern es treten auch anhaltende Ausfallserscheinungen auf, welche z. B. die kognitiven Leistungen, insbesondere Gedächtnisleistungen, beeinträchtigen. Dies kann auch zu sporadischen oder anhaltenden Demenz-Zuständen führen. Auch die bereits genannten psychiatrischen Symptome, wie z. B. ein allgemeiner Kontrollverlust, können sich chronisch manifestieren. Diese chronischen Folgeerscheinungen des Alkoholmißbrauchs - die bei anderen Suchtmittelabhängigkeiten in ähnlicher Weise auftreten - stellen ein erhebliches Hindernis für eine erfolgreiche Durchführung von Entwöhnungstherapien dar. So ist bekannt, daß der durch chronischen Alkoholmißbrauch bedingte Kontrollverlust eine Abstinenz-Unfähigkeit des betroffenen Alkoholkranken bewirkt. Dies ist die Hauptursache dafür, daß selbst entwöhnte Alkoholiker zu Rückfällen neigen, mit meist schwerwiegenden Folgen. Aus dieser Beobachtung wurde der Grundsatz abgeleitet, daß ein "kontrolliertes Trinken" bei Abhängigen nicht möglich ist.

Es ist ferner bekannt, daß es hinsichtlich des Rauschmittelkonsumverhaltens große individuelle Unterschiede gibt, aufgrund derer man beispielsweise Alkoholiker in unterschiedliche Trinker-Kategorien einteilt.

Bei bestimmten Alkohol-Konsumenten besteht das Problem, daß nach Überschreiten einer bestimmten individuellen Schwellendosis rasch ein allgemeiner Kontrollverlust mit den oben erwähnten negativen Begleiterscheinungen eintritt. Die betroffenen Personen sind meist nicht in der Lage, das Erreichen ihrer individuellen Schwellendosis oder überhaupt ihr persönliches Rückfall-Risiko rechtzeitig zu erkennen. Aufgrund des dadurch bewirkten Kontrollverlustes, der häufig zu weiterem, exzessiven Alkoholgenuß führt, werden solche Personen oft als "dangerous drinkers" bezeichnet. Häufig handelt es sich dabei um Personen, die bereits Entzugstherapien hinter sich haben und auf diese Weise rückfällig werden.

Bekanntermaßen hat der durch chronischen Suchtmittelmißbrauch verursachte Kontrollverlust, wie auch die Beeinträchtigung der Gedächtnisleistungen (bis hin zur Demenz) für den Betroffenen wie auch für seine Umgebung oft weiterreichende Folgen, wie z. B. Unfähigkeit zur Berufsausübung, Unfähigkeit zur Strukturierung des Tagesablaufs, Unfähigkeit zur Aufnahme und Pflege sozialer Kontakte und daraus folgend soziale Ausgrenzung.

Diese Ausfallserscheinungen, z. B. die Beeinträchtigung der kognitiven Leistungen, dauern selbst nach einer erfolgreich abgeschlossenen Entzugstherapie oft noch an. Weitere bei Alkoholmißbrauch oder bei Mißbrauch anderer Suchtmittel auftretende psychiatrische oder zerebrale Störungen sind z. B.: Wahrnehmungs- oder Sinnestäuschungen, Amnesie, Bewußtseinsveränderungen, formale Denkstörungen, Merkfähigkeitsstörungen, Wahnvorstellungen, Konfabulationen, Desorientiertheit, Erregungszustände.

Die beschriebenen psychiatrischen oder zerebralen Störungen, insbesondere eine Beeinträchtigung der kognitiven Leistungen oder Demenz, können auch infolge der Zuführung bzw. des Ge- oder Mißbrauchs anderer Sucht- und Rauschmittel, insbesondere dur Gebrauch oder Mißbrauch von halluzinogenen Substanzen auftreten. Sie können ferner durch Zuführung oder Aufnahme anderer Wirkstoffe, z. B. wie Umweltgiften (PCB, Dioxine, Furane, Pentachlorphenol, Quecksilber- und Bromverbindungen, Amalgam, chlorierte Kohlenwasserstoffe wie bestimmte Lösungsmittel) verursacht worden sein.

Es bestand daher die Aufgabe, die infolge eines gelegentlichen oder chronischem Gebrauchs oder Mißbrauchs von halluzinogenen Substanzen, bei einer akuten Vergiftung oder bei chronischer Einwirkung von Umweltgiften auftretenden Verlust kognitiver Fähigkeiten zu beseitigen oder zumindest abzumildern.

Aus US 4,663,318 ist ein Verfahren zur Behandlung der Alzheimerschen Erkrankung und verwandter Demenzerkrankungen bekannt, bei dem eine therapeutisch wirksame Mange an Galanthamin oder an einem seiner pharmazeutisch annehmbaren Salze verabreicht werden soll. Die als therapeutisch wirksam angegebene Menge Galanthamin soll bei parenteraler Verabreichung zwischen 5 und 1000 mg betragen. Ferner wird in diesem Dokument angegeben, daß eine radioaktiv markierte Version des Galanthamins als Mittel zur Diagnose der Alzheimerschen Erkrankung verwendet werden kann.

Mit der Offenlegungsschrift DE 195 09 663 A1 wird ein Verfahren zur Isolierung von Galanthamin aus biologischem Material beschrieben, vorzugsweise aus den Zwiebeln landwirtschaftlich angebauter Amaryllidaceenarten. Das mit dem Verfahren isolierte Galanthamin soll bei der Behandlung der Alzheimerschen Erkrankung, der Alkohol- und Nikotinabhängigkeit sowie der Therapie des Engwinkelglaukoms eingesetzt werden.

WO 00/30446 A1 offenbart die Verwendung von zentralnervös wirkenden Inhibitoren der Acetylcholinesterase zur Behandlung der Alzheimerschen Erkrankung. Hierbei soll, der Inhibitor der Acetylcholinesterase, vorzugsweise Galanthamin, Lycoramin oder Rivastigmin, mit Hilfe einer Zubereitung verabreicht werden, die einen verzögerten Wirkungseintritt des Inhibitors ermöglicht. Als dafür geeignete Darreichungsformen werden Tabletten, Kapseln, Caplets oder dergleichen angegeben.

WO 92/20328 A offenbart Verbesserungen bei der Behandlung von Patienten mit Benzodiazepinen durch Verabreichung von Inhibitoren der Cholinesterase, zu denen auch Galanthamin zählt. Die Gabe von Galanthamin oder einem anderen Cholinesterase-Inhibitor kann den sedativen, hypnotischen oder das Atemzentrum lähmenden Nebenwirkungen von Benzodiazepinen entgegenwirken. Die erwünschten Wirkungen der Benzodiazepine werden durch Verabreichung von Galanthamin jedoch nicht beeinträchtigt. Die Inhibitoren der Cholinesterase können auch zeitlich später als die Benzodiazepine verabreicht werden, insbesondere wenn die zentralen Symptome als Folgeerscheinung einer Überdosierung, beispielsweise aufgrund eines nicht bestimmungsgemäßen Mißbrauchs der Benzodiazepine als Suchtmittel, auftreten.

Stuntz, P. M. et al. (Cholinergic stimulation enhances cognitive performance following cingulate cortex lesions in rats; SCOCIETY FOR NEUROSCIENCE ABSTRACTS, Bd. 26, Nr. 1-2, 2000, Abstract No. 753.11) beschreiben, wie parenteral verabreichtes Galanthamin zu einer Verbesserung der kognitiven Ausfälle bei Ratten führt, bei denen 14 Tage vor der Verabreichung von Galanthamin chemisch Läsionen in der Hirnrinde, die zumindest cholinerge und noradrenerge Neuronen betreffen, hervorgerufen worden waren. Zwar verbesserte die Stimulation des cholinergen Neurotransmittersystems die Wiedererlangung kognitiver Fähigkeiten bei den Versuchstieren, der noradrenerge Agonist Protriptylin war in dieser Hinsicht jedoch wirkungslos.

Zur Lösung dieses Problems wird vorgeschlagen, zur Behandlung von Personen, die an derartigen Folgeerscheinungen leiden, den Wirkstoff Galanthamin zu verwenden. Durch Verabreichung von Galanthamin kann bewirkt werden, daß der eingetretene Verlust kognitiver Fähigkeiten zumindest teilweise aufgehoben bzw. rückgängig gemacht wird, so daß die genannten Fähigkeiten nach und nach wiedererlangt werden.

Gegenstand der vorliegenden Erfindung ist daher die Verwendung von Galanthamin, als freie Base oder als Säureadditionssalz, zur Herstellung eines Arzneimittels zur Behandlung von kognitiven Störungen, die als Folgeerscheinung eines gelegentlichen oder chronischen Gebrauchs oder Mißbrauchs von halluzinogenen Substanzen, oder bei einer akuten Vergiftung, oder bei chronischer Einwirkung von Umweltgiften beim Menschen oder anderen Wirbeltieren auftreten.

Die Erfindung beruht auf der überraschenden Beobachtung, daß im Tierversuch bei Ratten durch Verabreichung von Galanthamin eine Wiedererlangung der kognitiven Fähigkeiten herbeigeführt werden konnte. Bei nicht behandelten Kontrolltieren trat diese Wiederherstellung nicht oder erst wesentlich später ein. Weiterhin konnte in klinischen Studien mit chronisch abhängigen Alkoholikern gezeigt werden, daß durch Galanthamin die kognitiven Leistungen von Probanden allgemein verbessert wurden. Ein weiterer Vorteil ist dabei, daß insbesondere eine verbesserte Kontrolle über das Suchtverhalten ermöglicht wurde, so daß das Rückfall-Risiko verringert wird.

Galanthamin (4a,5,9,10,11,12-Hexahydro-3-methoxy-11-methyl-6-H-benzofuro-(3a,3,2-ef)-(2)-benzazepin-6-ol) ist ein tetracyclisches Alkaloid, das bei bestimmten Pflanzen, insbesondere bei Amaryllidaceen, vorkommt. Es kann aus diesen Pflanzen mittels bekannter Verfahren gewonnen werden (z. B. gemäß DE 195 09 663 A1 oder DE-PS 1 193 061) oder auf synthetischem Wege (z. B. Kametani et al., Chem. Soc. C. 6, 1043-1047 (1971) oder Shimizu et al., Heterocycles 8, 277-282 (1977)) hergestellt werden.

Aufgrund seiner pharmakologischen Eigenschaften wird Galanthamin zur Gruppe der reversibel wirkenden Cholinesterasehemmstoffe gezählt. Galanthamin wird zur Behandlung der Poliomyelitis, der Alzheimerschen Krankheit und verschiedener Erkrankungen des Nervensystems eingesetzt, sowie zur Behandlung des Engwinkelglaukoms.

Ferner wurde vorgeschlagen, Galanthamin zur Behandlung der Nicotin- bzw. Alkoholabhängigkeit zu verwenden (DE 43 01 782 C1, DE 40 10 079 A1), wobei Galanthamin bei Süchtigen das Verlangen nach Nicotin bzw. Alkohol unterdrücken soll.
Eine Anwendung zur Behandlung der durch chronischen Mißbrauch von Rauschmitteln, insbesondere Alkohol, verursachten Symptome oder Folgeerscheinungen wie Kontrollverlust, Verlust kognitiver Fähigkeiten etc., ist bisher nicht beschrieben worden.

Nach der Erfindung kann Galanthamin sowohl in Form seiner freien Base als auch als Säureadditionssalz zur Herstellung des Arzneimittels verwendet werden; als Salze werden Galanthaminhydrochlorid und Galanthaminhydrobromid bevorzugt.

Galanthamin wird vorzugsweise in einem Arzneimittel bereitgestellt, welches den Wirkstoff in Anteilen von 0,1 bis 90 Gew.-%, besonders bevorzugt in Anteilen von 2 bis 20 Gew.-%, enthält, jeweils berechnet als freies Galanthamin. Die erfindungsgemäßen galanthaminhaltigen Arzneimittel können darüber hinaus Hilfsstoffe, Trägerstoffe, Stabilisatoren etc. in den dem Fachmann bekannten Mengen enthalten. Die täglich verabreichte Dosis liegt vorzugsweise im Bereich von 0,1 bis 100 mg, insbesondere von 10 bis 50 mg.

Die Arzneimittel, welche gemäß vorliegender Erfindung zur Verabreichung von Galanthamin verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.

Diese Aufzählung ist nicht abschließend; die in Frage kommenden physiologisch unbedenklichen Substanzen sind dem Fachmann bekannt.

Die Verabreichung von Galanthamin kann oral oder parenteral erfolgen. Für die orale Verabreichung können bekannte Darreichungsformen wie Tabletten, Dragees oder Pastillen verwendet werden. Daneben kommen auch flüssige oder halbflüssige Darreichungsformen in Betracht, wobei der Wirkstoff als Lösung oder Suspension vorliegt. Als Löse- oder Suspendierungsmittel können Wasser, wässrige Medien oder pharmakologisch unbedenkliche Öle (vegetalische oder Mineralöle) verwendet werden. Vorzugsweise sind die Galanthamin enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann die Verabreichung von Galanthamin nach der Erfindung auch auf parenteralem Wege erfolgen. Hierzu können transdermale oder transmucosale Darreichungsformen besonders vorteilhaft für die erfindungsgemäße Verabreichung von Galanthamin verwendet werden, insbesondere klebende transdermale therapeutische Systeme (Wirkstoffpflaster). Diese ermöglichen es, den Wirkstoff über einen längeren Zeitraum in kontrollierter Weise über die Haut an den zu behandelnden Patienten abzugeben.

Derartige Systeme weisen üblicherweise eine wirkstoffhaltige, haftklebende Polymermatrix auf, die auf der hautfernen Seite von einer wirkstoffundurchlässigen Rückschicht bedeckt ist, und deren klebende, wirkstoffabgebende Oberfläche vor der Applikation mit einer ablösbaren Schutzschicht bedeckt ist. Die Herstellung solcher Systeme und die dabei verwendbaren Grundstoffe und Hilfsstoffe sind dem Fachmann grundsätzlich bekannt; beispielsweise wird der Aufbau solcher transdermaler therapeutischer Systeme in den deutschen Patenten DE 33 15 272 und DE 38 43 239 oder in den US-Patenten 4 769 028, 5 089 267, 3 742 951, 3 797 494, 3 996 934 und 4 031 894 beschrieben.

Durch die vorliegende Erfindung wird es möglich, bestimmte Begleit- oder Folgeerscheinungen eines gelegentlichen oder chronischen Gebrauchs oder Mißbrauchs von halluzinogenen Substanzen zu behandeln, wodurch das Allgemeinbefinden dieser Patienten verbessert wird und die soziale Wiedereingliederung dieser chronisch Suchtmittelgeschädigten unterstützt wird. Außerdem werden durch die vorgeschlagene Behandlung mit Galanthamin die Erfolgsaussichten von Entzugstherapien verbessert und das Rückfallrisiko vermindert. Die Behandlung fördert zudem die soziale Reintegration der betroffenen Personen.

Die beschriebenen Beeinträchtigungen der kognitiven Leistungen oder Demenz können auch infolge der Zuführung oder Aufnahme anderer Wirkstoffe, z. B. wie Umweltgiften (PCB, Dioxine, Furane, Pentachlorphenol, Quecksilber- und Bromverbindungen, Amalgam, chlorierte Kohlenwasserstoffe wie bestimmte Lösungsmittel) verursacht worden sein.

Unter Sucht- und Rauschmitteln werden grundsätzlich alle psychotropen Substanzen, seien sie fest, flüssig, dampf- oder gasförmig, verstanden, bei deren einmaligem, gelegentlichem, häufigem oder chronischem Gebrauch oder Mißbrauch Krankheitserscheinungen der genannten Art auftreten.

Insbesondere bezieht sich die Erfindung auf halluzinogene Substanzen aus der Gruppe der folgenden Psychopharmaka: Neuroleptika, Antidepressiva, Tranquilizer (insbesondere Benzodiazepine), Antipsychotika, Hypnotika, Psychostimulantien (insbesondere Amphetamine, "Modedrogen" wie z. B. "Ecstacy", "Speed" mit unstandardisierten Wirkstoffgemischen), natürliche psychotrope Drogen und Substanzen sowie synthetische Derivate davon (z. B. auf der Basis von Johanniskraut, Baldrian, Hopfen, Melisse, Lavendel, Kava-Kava, Absinth, weißer Stechapfel (Datura stramonium), Engelstrompete (Brugsmania spp.), Tollkirsche (Atropa belladonna), GHB (gamma-Hydroxybuttersäure); ferner Tetrahydrocannabiol (THC) und THC-haltige Rauschmittel wie Marihuana und Haschisch, weiterhin Kokain, "Crack", LSD, Psilocybin, Mescalin, Stoffgemische wie in Opium und Absinth, Morphium und Morphinderivate wie Heroin, Codein, Methadon, Scopolamin und Hyoscyamin), sowie bestimmte organische Lösungsmittel wie Aceton, Benzin, Äther, Toluol, Trichloräthylen, Trichlormethan, sowie sonstige Gase und Gasgemische, welche "geschnüffelt" bzw. eingeatmet werden, z. B. Distickstoffmonoxid.

Auch bei der bestimmungsgemäßen Anwendung einiger der vorstehend genannten Substanzen, die für Therapiezwecke eingesetzt werden, kann es im Rahmen einer ärztlich verordneten Therapie, insbesondere bei wiederholter oder über längere Zeit erfolgender Verabreichung, zu den genannten kognitive Störungen, z. B., zerebrale Ausfallserscheinungen, psychiatrische Symptome etc., kommen.

Es wird deshalb vorgesehen, zur Behandlung solcher Störungen, welche durch die bestimmungsgemäße oder mißbräuchliche Anwendung der vorstehend genannten Substanzen verursacht wurden, den Wirkstoff Galanthamin zu verabreichen. Das gleiche gilt für Intoxikationen durch die genannten Umweltgifte.

Auch bei akuten Vergiftungsfällen (z. B. Chemie-Unfälle) sowie bei chronischer Gifteinwirkung (z. B. Umweltgifte wie Holzschutzmittel, halogenierte Biphenyle, Dioxine, Furane, Pentachlorphenol, Quecksilber- und Bromverbindungen, Quecksilber, Amalgame, halogenierte Kohlenstoff- und Kohlenwasserstoffverbindungen, insbesondere chlorierte Kohlenwasserstoffe, halogenierte Biphenyle, Tributylzinn, Chrom-(VI)-haltige Holzschutzmittel etc.) werden zerebrale Störungen oder psychiatrische Symptome, z. B. Gedächtnisverlust oder Denkstörungen, bei den betroffenen Personen beobachtet. Die erfindungsgemäße Verwendung von Galanthamin erstreckt sich deshalb auch auf Medikamente zur Behandlung von Personen, welche auf die vorstehend genannte Art durch Gifteinwirkung beeinträchtigt wurden.

## Patentansprüche

1. Verwendung von Galanthamin, als freie Base oder als Säureadditionssalz, zur Herstellung eines Arzneimittels zur Behandlung von kognitiven Störungen, die als Folgeerscheinung
- eines gelegentlichen oder chronischen Gebrauchs oder Mißbrauchs von halluzinogenen Substanzen, oder
- bei einer akuten Vergiftung, oder
- bei chronischer Einwirkung von Umweltgiften
beim Menschen oder anderen Wirbeltieren auftreten.

2. Verwendung nach Anspruch 1, **dadurch gekennzeichnet, daß** das Arzneimittel zur Behandlung eines allgemeinen Kontrollverlustes, des Gedächtnisverlustes oder einer Beeinträchtigung der Gedächtnisleistung, des Denkvermögens, von Demenz, von Wahrnehmungsstörungen, oder einer Beeinträchtigung der Bewegungskoordination verwendet wird.

3. Verwendung nach Anspruch 1 oder 2, **dadurch gekennzeichnet, daß** das genannte Arzneimittel den Wirkstoff Galanthamin in Anteilen von 0,1 bis 90 Gew.-%, vorzugsweise 2 bis 20 Gew.-%, berechnet als freies Galanthamin, enthält.

4. Verwendung nach einem der Ansprüche 1 bis 3, **dadurch gekennzeichnet, daß** das genannte Arzneimittel eine Depotwirkung aufweist.

5. Verwendung nach einem der Ansprüche 1 bis 4, **dadurch gekennzeichnet, daß** das genannte Arzneimittel ein oral verabreichbares Arzneimittel ist.

6. Verwendung nach einem der Ansprüche 1 bis 4, **dadurch gekennzeichnet, daß** das genannte Arzneimittel ein parenteral verabreichbares Arzneimittel ist.

7. Verwendung nach Anspruch 6, **dadurch gekennzeichnet, daß** das genannte Arzneimittel ein transdermal verabreichbares Arzneimittel ist.

## Claims

1. Use of galanthamine, as free base or as acid addition salt, for the manufacture of a medicament for the treatment of cognitive disorders occurring as sequelae
- to occasional or chronic use or abuse of hallucinogenic substances, or
- in cases of acute poisoning, or
- in cases of chronic exposure to environmental poisons in humans or other vertebrates.

2. Use according to Claim 1, **characterized in that** said medicament is used to treat general loss of control, memory loss or impairment of memory performance, cognitive ability, dementia, perception disturbances, or impairment of muscular coordination.

3. Use according to claim 1 or 2, **characterized in that** said medicament contains the active agent galanthamine in proportions of from 0.1 to 90% by weight, preferably 2 to 20% by weight, calculated as free galanthamine.

4. Use according to any one of Claims 1 to 3, **characterized in that** said medicament has a depot effect.

5. Use according to any one of Claims 1 to 4, **characterized in that** said medicament is a medicament that can be administered orally.

6. Use according to any one of Claims 1 to 4, **characterized in that** said medicament is a medicament that can be administered parenterally.

7. Use according to Claim 6, **characterized in that** said medicament is a medicament that can be administered transdermally.

## Revendications

1. Utilisation de galanthamine, comme base libre ou comme sel d'addition d'acide, pour la fabrication d'un médicament pour le traitement des désordres cognitifs qui apparaissent chez l'homme ou chez d'autres vertébrés à la suite
- d'une utilisation ponctuelle ou chronique ou d'un abus de substances hallucinogènes, ou
- d'un empoisonnement aigu, ou
- de l'effet chronique de pollutions de l'environnement.

2. Utilisation selon la revendication 1, **caractérisée en ce que** le médicament est utilisé pour le traitement d'une perte de contrôle généralisée, de la perte de mémoire ou d'une atteinte de la capacité mémorielle ou de la faculté de penser, de la démence, des désordres de la perception, ou d'une atteinte de la coordination motrice.

3. Utilisation selon la revendication 1 ou 2, **caractérisée en ce que** le médicament mentionné contient la substance active galanthamine à des proportions de 0,1 à 90 % en poids, de préférence de 2 à 20 % en poids, calculée sous forme de galanthamine libre.

4. Utilisation selon l'une des revendications 1 à 3, **caractérisée en ce que** le médicament mentionné présente un effet retard.

5. Utilisation selon l'une des revendications 1 à 4, **caractérisée en ce que** le médicament mentionné est un médicament administrable par voie orale.

6. Utilisation selon l'une des revendications 1 à 4, **caractérisée en ce que** le médicament mentionné est un médicament administrable par voie parentérale.

7. Utilisation selon la revendication 6, **caractérisée en ce que** le médicament mentionné est un médicament administrable par voie transdermique.
